# EUROPEAN PATENT APPLICATION

(11) **EP 0 651 595 A1**
(43) Date of publication of application: **03.05.1995**
(21) Application number: 94203116.2
(22) Date of filing: 26.10.1994
(51) Int. Cl.: H05F 7/00, A61N 1/16

(54) **Process and associated device for the neutralisation of the effects of electromagnetic fields within open areas and/or volumes**

(30) Priority: 27.10.1993 IT MI932291
(71) Applicant: Ciccolo, Enza, Milano (IT)
(72) Inventor: Ciccolo, Enza, Milano (IT)
(74) Representative: Raimondi, Alfredo, Dott. Ing. Prof.

(57) **Abstract**

Device for neutralizing the effects of electromagnetic fields inside areas and/volumes in which said fields occur, consisting of a pyramid (1) having as its base a rhombus (2), the greater diagonal (D1) and smaller diagonal (d2) of which are respectively of a length such as to give rise to a so-called golden ratio D1/d2, and also of a height (h) such as to give rise to a ratio h/D1 also of the golden type, there also being provided at least one pair of arms (3) and (4) respectively parallel to the greater diagonal (D1) and to the smaller diagonal (d2) and respectively joining pairs of opposite edges (1a,1c,1b,1d) of the pyramid (1), said arms (3,4) supporting in the region of said edges respective containers (3N,3S,4E,4W) designed to contain a predetermined quantity of a liquid and intersecting in the region of a further, central, container (5) arranged along the height of the pyramid.

## Description

The present invention relates to a process and an associated device for neutralizing the effects of electromagnetic fields inside areas and/or volumes inside which said fields occur.

It is known that the presence of electromagnetic fields of any origin inside areas and/or volumes gives rise to environmental pollution phenomena which are harmful both for any vegetation as well as for fauna and man.

It is known, for example, that the prolonged exposure of the eyes to the rays of a video terminal such as that of a computer is harmful for the eyes themselves and it is also known that agriculture fields exposed to the electromagnetic waves of high-voltage lines are affected, with a reduction in the productivity of the crops.

The technical problem which is posed, therefore, is that of neutralizing the effects of electromagnetic fields present inside open areas and/or volumes such as closed environments in order to reduce significantly the harmful effects of said electromagnetic fields.

These results are obtained by the present invention which envisages a process for neutralizing the effects of electromagnetic fields inside areas and/or volumes in which said fields occur, which comprises the following phases:
- determination of the direction of North-South orientation of the area "A" subject to the electromagnetic field,
- corresponding determination of the direction of East-West orientation of the same area,
- determination of a segment arranged along the orientation N-S and of a segment arranged along E-W such that they constitute respectively the greater diagonal D2 and the smaller diagonal d3 of a rhombus 6 inside which the area subject to neutralization treatment is inscribed and such that their ratio is of the golden type;
- arrangement, in the region of each vertex N,E,S,W, of the above-defined rhombus, of a pyramid device according to the present invention (1N,1E,1S,1W), each oriented with the larger diagonal parallel to the direction N-S,
- arrangement, in the containers (3N,3S,4E,4W) corresponding to the respective cardinal point at which they are located, of a liquid, vibration of which resonates with the specific frequency of the cardinal point in question, i.e.:
   North: frequencies C = 10 Hz ; E = 40 Hz
   South: frequencies A = 2.5 Hz ; G = 160 Hz
   East: frequency D = 20 Hz
   West: frequencies B = 5 Hz ; F = 80 Hz,
- arrangement of a further pyramid device (1C) at the centre of the above-defined rhombus,
- arrangement, in the central container (5C) of said pyramid (1C), of a predetermined quantity of liquid, the vibration frequency of which resonates with a reference frequency D = 20 Hz.

The present invention also relates to a device for neutralizing the effects of electromagnetic fields inside areas and/or volumes in which said fields occur, consisting of a pyramid having as its base a rhombus, the larger and smaller diagonals of which are respectively of a length such as to give rise to a so-called golden ratio and also of a height such as to give rise to a ratio also of the golden type, there being provided moreover at least one pair of arms respectively parallel to the larger diagonal and to the smaller diagonal and respectively joining pairs of opposite edges of the pyramid, said arms supporting in the vicinity of said edges respective containers designed to contain a predetermined quantity of a liquid and intersecting in the region of a further, central, container arranged along the height of the pyramid.

Further details may be obtained from the following description, with reference to the accompanying drawings, in which:
Figure 1 shows a perspective view of the device for neutralizing electromagnetic fields according to the invention, and
Figure 2 shows a diagram illustrating application of the process to a predetermined area.

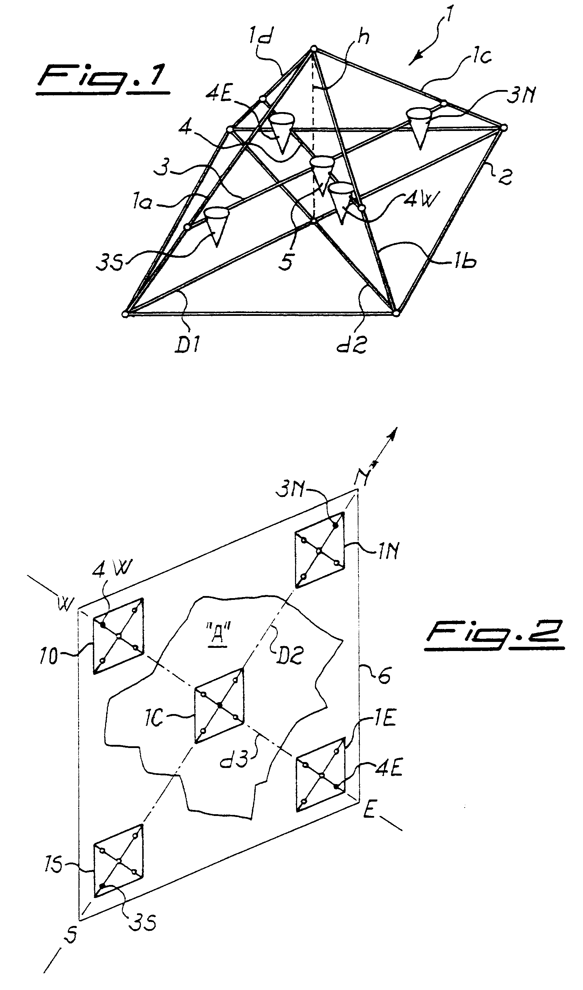

As shown in Figure 1, the device according to the invention consists of a pyramid 1 made of conductive material such as copper, zinc or silver/gold having as its base a rhombus 2, the greater diagonal D1 and smaller diagonal d2 of which are respectively of a length such as to give rise to a so-called golden ratio D1/d2, i.e. such that the value of the ratio is equal to the square root of the ratio between the difference of the squares of D1 and d2 and the square of d2.

The pyramid also has a height h such as to give rise to a ratio h/D1 also of the golden type, i.e. such that the value of the ratio is equal to the square root of the ratio between the difference of the squares of h and D1 and the square of h.

Moreover, inside the pyramid there are provided arms 3 and 4 respectively parallel to the greater diagonal D1 and smaller diagonal d2 and joining the opposite edges 1a, 1c and 1b, 1d, respectively.

Said arms 3 and 4 support, in the vicinity of said edges, respective containers 3N, 3S and 4E, 4W designed to contain a predetermined quantity of a liquid described in more detail below.

Moreover, it is envisaged that said arms 3 and 4 are connected to a further liquid container 5 arranged at the point of intersection of the arms themselves, located along the height of the pyramid.

The said liquid contained in the associated container is called active water and consists of particular types of water having vibration frequences resonating with particular reference frequences which are typical of the resonance of biological tissue in light and can be measured with instruments known as "GIR" or microlasers.

Said reference frequences are seven in number and identified by a letter of the alphabet and by a value in Hz.

The processes for identifying and reproducing said types of water have already being dealt with in previous patent applications in the name of the same Applicant and will not be described further, except in order to specify the particular frequencies to be used in relation to the neutralization process which will be described below.

With reference to Figure 2, a description is given below of the process for neutralizing the effects of electromagnetic fields ascertained beforehand by means of verification of the type of alteration induced by the magnetic field on the specific resonance frequencies of each cardinal point in relation to specific predetermined reference values; the process comprises the following phases:
- determination of the direction of North-South orientation of the area "A" subject to the electromagnetic field,
- corresponding determination of the direction of East-West orientation of the same area,
- determination of a segment arranged along the orientation N-S and of a segment arranged along E-W such that they constitute respectively the greater diagonal D2 and smaller diagonal d3 of a rhombus 6 inside which the area subject to neutralization treatment is inscribed and such that their ratio is of the golden type as defined above,
- arrangement, in the region of each vertex N,E,S,W of the above-defined rhombus, of a pyramid device according to the present invention 1N,1E,1S,1W, respectively, all oriented with the greater diagonal parallel to the direction N-S,
- arrangement, in the containers 3N,3S,4E,4W corresponding to the respective cardinal point at which they are located, of a liquid, vibration of which resonates with the specific frequency of the cardinal point in question, i.e.:
   North: frequencies C = 10 Hz ; E = 40 Hz
   South: frequencies A = 2.5 Hz ; G = 160 Hz
   East: frequency D = 20 Hz
   West: frequencies B = 5 Hz ; F = 80 Hz,
- arrangement of a further pyramid device 1C at the centre of the above-defined rhombus 6,
- arrangement in the central container 5C of said pyramid 1C, of a predetermined quantity of liquid, the vibration frequency of which resonates with a reference frequency D = 20 Hz.

Preferably the neutralization cycle has a duration of 28 days which can be calculated from any day on which the cycle is started; it is preferred, however, to start the treatment cycle on the third day preceding the day on which there is a full moon; correspondingly, the quantity of liquid to be arranged in the associated containers must be such that it lasts for the entire period even with respect to the different atmospheric conditions, although the process is not affected by any periodic topping-up of the said liquids.

At the end of the 28-day treatment period the neutralization effects obtained can be verified by checking again the individual frequencies of each single cardinal point and repeating, if necessary, neutralization cycles in order to maintain the renewed frequency equilibrium obtained.

Many variants may be introduced during realization of the component parts of the invention, without thereby departing from the protective scope of the present invention as defined in the claims which follow.

## Claims

1. Process for neutralizing the effects of electromagnetic fields inside areas and/or volumes in which said fields occur, characterized in that it comprises the following phases:
- determination of the direction of North-South orientation of the area "A" subject to the electromagnetic field,
- corresponding determination of the direction of East-West orientation of the same area,
- determination of a segment arranged along the orientation N-S and of a segment arranged along E-W such that they constitute respectively the greater diagonal D2 and smaller diagonal d3 of a rhombus 6 inside which the area subject to neutralization treatment is inscribed and such that their ratio is of the golden type;
- arrangement, in the region of each vertex N,E,S,W of the above-defined rhombus, of a pyramid device according to the present invention (1N,1E,1S,1W), each oriented with the greater diagonal parallel to the direction N-S,
- arrangement in the containers (3N,3S,4E,4W) corresponding to the respective cardinal point at which they are located, of a liquid, vibration of which resonates with the specific frequency of the cardinal point in question, i.e.:
North: frequencies C = 10 Hz ; E = 40 Hz
South: frequencies A = 2.5 Hz ; G = 160 Hz
East: frequency D = 20 Hz
West: frequencies B = 5 Hz ; F = 80 Hz
- arrangement of a further pyramid device (1C) at the centre of the above-defined rhombus (6),
- arrangement, in the central container (5C) of said pyramid (1C), of a predetermined quantity of liquid, the vibration frequency of which resonates with a reference frequency D = 20 Hz.

2. Process according to Claim 1 characterized in that the neutralization cycle has a duration of 28 days.

3. Process according to Claim 1 characterized in that the neutralization cycle starts on the third day preceding the day on which there is a full moon.

4. Process according to Claim 1 characterized in that the ratio between the diagonals (D2,d3) is of the golden type, i.e. such that the value of the ratio (D2/d3) is equal to the square root of the ratio between the difference of the squares of D2 and d3 and the square of d3.

5. Device for neutralizing the effects of electromagnetic fields inside areas and/or volumes in which said fields occur, characterized in that it consists of a pyramid (1) having as its base a rhombus (2), the greater diagonal (D1) and smaller diagonal (d2) of which are respectively of a length such as to give rise to a so-called golden ratio D1/d2, and also of a height (h) such as to give rise to a ratio h/D1 also of the golden type, there also being provided at least one pair of arms (3) and (4) respectively parallel to the greater diagonal (D1) and to the smaller diagonal (d2) and respectively joining pairs of opposite edges (1a,1c,1b,1d) of the pyramid (1) said arms (3,4) supporting in the vicinity of said edges respective containers (3N, 3S, 4E, 4W) designed to contain a predetermined quantity of a liquid and intersecting in the region of a further, central, container (5) arranged along the height of the pyramid.

6. Device according to Claim 5, characterized in that said golden ratio between the greater diagonal (D1) and smaller diagonal (d2) is such that the value of the ratio is equal to the square root of the ratio betweeen the difference of the squares of D1 and d2 and the square of d2.

7. Device according to Claim 5, characterized in that said pyramid is made of conductive material.

8. Device according to Claim 5, characterized in that said containers are made of a natural material such as clay and the like.

9. Device according to Claim 5, characterized in that said liquid introduced into each container at the vertices is a type of water, the vibration frequency of which resonates with the frequency of the cardinal point corresponding to the associated vertex.

10. Device according to Claim 5, characterized in that said liquid introduced into the central container is a type of water, the vibration frequency of which resonates with the frequency D = 20 Hz.
